(19) **Europäisches Patentamt European Patent Office Office européen des brevets**

(11) **EP 1 656 927 B1**

(12) **FASCICULE DE BREVET EUROPEEN**

(45) Date de publication et mention
de la délivrance du brevet:
**16.09.2009 Bulletin 2009/38**

(51) Int Cl.:
*A61K 8/19* (2006.01)          *A61K 8/25* (2006.01)
*A61K 8/26* (2006.01)          *A61K 8/29* (2006.01)
*A61Q 1/06* (2006.01)          *A61Q 1/12* (2006.01)
*A61Q 19/00* (2006.01)

(21) Numéro de dépôt: **05300913.0**

(22) Date de dépôt: **10.11.2005**

(54) **Composition de maquillage à effet éclaircissant et/ou camouflant**

Kosmetische Zusammensetzung mit aufhellender und/oder abdeckender Wirkung

Cosmetic composition having a lightening and/or concealing effect

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **12.11.2004 FR 0452611**

(43) Date de publication de la demande:
**17.05.2006 Bulletin 2006/20**

(73) Titulaire: **L'ORÉAL**
**75008 Paris (FR)**

(72) Inventeur: **Cassin, Guillaume**
**91140, VILLEBON SUR YVETTE (FR)**

(74) Mandataire: **Le Coupanec, Pascale A.M.P. et al Nony & Associés,**
**3 rue de Penthièvre**
**75008 Paris (FR)**

(56) Documents cités:
**EP-A- 0 701 810          WO-A- 00/51551**
**FR-A- 2 673 372          US-A- 6 117 435**

Remarques:
Le dossier contient des informations techniques présentées postérieurement au dépôt de la demande et ne figurant pas dans le présent fascicule.

**Description**

La présente invention concerne principalement des compositions cosmétiques destinées au maquillage et/ou au soin de la peau et des lèvres et notamment utiles pour procurer sur celles-ci un effet éclaircissant, unifiant, voire camouflant à l'égard des imperfections cutanées.

Il est fréquent que des personnes présentent des dyschromies comme des taches pigmentaires, de la couperose ou des cernes au niveau de la peau, notamment du visage, souhaitent estomper ces défauts cutanés. De même, des personnes de peau colorée peuvent souhaiter éclaircir la teinte naturelle de leur peau.

En ce qui concerne plus particulièrement l'aspect éclaircissement de la peau, il est déjà connu d'utiliser des produits contenant des actifs blanchissants à l'image par exemple de l'hydroquinone. Toutefois, ces produits ont pour effet d'être relativement agressifs et par ailleurs de nécessiter un traitement prolongé avant obtention d'un résultat. Ils ne permettent donc pas de procurer un effet immédiat éclaircissant dès leur application au niveau de la peau.

Pour obtenir un effet éclaircissant et/ou camouflage immédiat, d'autres compositions alternatives ont déjà été proposées.

Une première alternative met à profit des composés fluorescents à l'image des azurants optiques. Toutefois, ces composés ne procurent un effet d'éclaircissement immédiat que dans conditions d'éclairage optimales généralement bien supérieures à celles procurées par la lumière naturelle ou un éclairage ordinaire.

Une seconde alternative vise à utiliser des pigments dits interférentiels, c'est-à-dire capables de procurer un reflet coloré différent selon leur angle d'observation. Malheureusement, ces compositions ont généralement pour inconvénient de procurer parallèlement un aspect brillant et donc non conforme à la carnation naturelle de la peau. Par ailleurs, ces pigments interférentiels sont généralement mis en oeuvre dans des conditions telles que la composition cosmétique correspondante procure un effet coloré et/ou couvrant trop imparfait et qui masque donc l'aspect naturel de la peau. De telles compositions sont notamment décrites dans les documents WO 01/51017 et US 5 690 916.

Une autre alternative décrite dans le document WO 04/045524 vise pour sa part à associer dans une même composition des pigments dits transparents avec des particules non interférentielles. Toutefois, ce type de composition ne s'avère pas totalement satisfaisant en terme de camouflage de dyschromies.

En conséquence, il demeure un besoin d'une composition cosmétique permettant de procurer un maquillage éclaircissant et unifiant tout en préservant la carnation naturelle de la peau en terme de couleur et/ou de brillance.

De manière inattendue, les inventeurs ont précisément mis en évidence qu'il était possible de formuler une telle composition cosmétique c'est-à-dire dotée d'un effet éclaircissant et/ou d'homogénéisation immédiat et prolongé du teint, sous réserve du choix de particules interférentielles particulières.

Plus précisément, la présente invention concerne, selon un premier aspect, une composition cosmétique pour le maquillage et/ou le soin de la peau et/ou des lèvres comprenant, dans un milieu physiologiquement acceptable, au moins des particules interférentielles particulières dont la taille moyenne en volume est inférieure à 40 $\mu$m, et au moins une charge particulière, ladite composition possédant en outre une clarté L* supérieure à 60 et une saturation C* inférieure à 5.

La présente invention concerne, selon un autre de ses aspects, une composition cosmétique pour le maquillage et/ou le soin de la peau et/ou des lèvres comprenant, dans un milieu physiologiquement acceptable, au moins des particules interférentielles particulières dont la taille moyenne en volume est inférieure à 40 $\mu$m et au moins une charge particulière, ladite composition possédant en outre une clarté L* supérieure à 75 et une saturation C* inférieure à 10.

Selon un autre de ses aspects, la présente invention concerne une composition cosmétique pour le maquillage et/ou le soin de la peau et/ou des lèvres comprenant, dans un milieu physiologiquement acceptable, au moins des particules interférentielles particulières dont la taille moyenne en volume est inférieure à 40 $\mu$m et au moins une charge choisie parmi des microparticules poreuses de silice, des particules hémisphériques creuses de silicone, et leurs mélanges, ladite composition possédant en outre une clarté L* supérieure à 60 et une saturation C* inférieure à 10.

Selon encore un autre de ses aspects, la présente invention concerne une composition cosmétique pour le maquillage et/ou le soin de la peau et/ou des lèvres comprenant, dans un milieu physiologiquement acceptable, au moins des particules interférentielles particulières dont la taille moyenne en volume est inférieure à 40 $\mu$m et au moins une charge, ladite charge étant en une quantité telle que le rapport pondéral charge/particules interférentielles varie de 0,3 à 2,5, en particulier de 0,5 à 1,2, ladite composition cosmétique possédant en outre une clarté L* supérieure à 60 et une saturation C* inférieure à 10.

Selon encore un autre de ses aspects, la présente invention concerne une composition cosmétique pour le maquillage et/ou le soin de la peau et/ou des lèvres comprenant, dans un milieu physiologiquement acceptable, au moins des particules interférentielles particulières dont la taille moyenne en volume est inférieure à 40 $\mu$m et au moins une charge particulière, pour conférer à ladite composition une variation de réflectance, mesurée pour une plage d'angles d'observation variant de 0 à 80° et à la longueur d'onde correspondant à la zone du spectre où la réflectance des particules interférentielles associées est la plus importante, telle que d'une part, la réflectance maximale mesurée au sommet du pic de réflexion est inférieure à 150 %, en particulier inférieure à 100 % et plus particulièrement inférieure à 75 %, et d'autre part, la réflectance minimale mesurée à la base du pic de réflexion est supérieure à 5 %, ladite composition possédant en outre une clarté L* supérieure à 60 associée à une saturation C* inférieure à 10.

Selon encore un autre de ses aspects, la présente invention vise l'utilisation cosmétique d'une composition telle que définie précédemment pour obtenir un effet d'éclaircissement et/ou d'homogénéisation de la peau et/ou des lèvres.

Selon encore un autre de ses aspects, la présente invention vise l'utilisation cosmétique d'une composition telle que définie précédemment pour le camouflage de dyschromies cutanées.

Selon encore un autre de ses aspects, la présente invention vise un procédé de maquillage de la peau et/ou des lèvres comprenant au moins l'application sur la peau et/ou les lèvres d'une composition telle que définie précédemment.

Les inventeurs ont mis ainsi en évidence qu'une sélection de particules interférentielles spécifiques et leur association à une charge, le cas échéant également particulière, permettait avantageusement d'obtenir un effet de maquillage doté d'une transparence et d'un effet éclaircissant améliorés au regard des compositions conventionnelles.

Au sens de la présente invention, "transparence" entend caractériser le fait que l'effet de maquillage et/ou de soin procuré par ladite composition au niveau de la peau n'affecte quasiment pas, voire pas du tout la carnation naturelle de celle-ci. Ainsi, les compositions selon l'invention ont pour avantage de ne pas conférer un aspect ni couvrant ni brillant, et donc de préserver au mieux la carnation naturelle de la peau sur laquelle elles sont appliquées. Les compositions selon l'invention se différencient à ce titre des compositions de fonds de teint classiques qui, d'une manière générale, affectent la carnation naturelle de la peau, en assombrissant ou modifiant significativement sa coloration naturelle ou à l'inverse en éclaircissant celle-ci de manière trop significative, ou encore en lui conférant un aspect trop brillant.

### Clarté L* et saturation C*

L'effet naturel procuré par les compositions selon l'invention est notamment caractérisé par des valeurs colorimétriques de clarté L* et de saturation C* mesurées dans l'espace colorimétrique CIE 1976.

Les mesures colorimétriques L* et C* peuvent être réalisées à l'aide d'un colorimétrique CHROMAMETER CR400® de MINOLTA.

Pour ce faire, la composition à tester est introduite dans un pot de volume de 15 ml (diamètre d'ouverture : 1,9 cm ; profondeur : 1,8 cm). La surface de la composition introduite est lissée par arasement avec une lame de verre. La cellule du colorimètre est alors mise en contact avec cette surface et les paramètres colorimétriques sont déterminés.

Pour information, la référence blanche fournie avec le colorimètre CHROMAMETER CR400® est caractérisée par les valeurs de clarté L* et de saturation C* suivantes :

$$L^* = 96,94 +/- 0,01, \; C^* = 2,83 +/- 0,01$$

Selon un premier mode de réalisation, les compositions selon l'invention se caractérisent par une clarté L* supérieure à 60 associée à une saturation C* inférieure à 5.

Selon un autre mode de réalisation, les compositions selon l'invention se caractérisent par une clarté L* supérieure à 75 associée à une saturation C* inférieure à 10.

Plus particulièrement, les compositions selon l'invention peuvent se caractériser par une clarté L* supérieure à 60, en particulier supérieure à 80, et plus particulièrement supérieure à 86.

Plus particulièrement, les compositions selon l'invention peuvent se caractériser par une saturation C* inférieure à 5, en particulier inférieure à 3, et notamment inférieure à 2.

D'une manière générale, les compositions conformes à ces exigences sont blanches dans la masse. Elles possèdent en outre moins de 5 % et plus particulièrement moins de 2 % en poids en oxyde(s) métallique(s).

### Particules interférentielles

Les particules interférentielles selon l'invention sont caractérisées par une taille moyenne en volume généralement inférieure à 40 $\mu$m, notamment allant de 0,5 à 40 $\mu$m, plus particulièrement inférieure à 30 $\mu$m, notamment inférieure à 20 $\mu$m, et en particulier inférieure à 15 $\mu$m, et mieux allant de 1 à 15 $\mu$m, mesurée par un granulomètre laser, tel que par exemple le Mastersizer 2000® de Malvernet ou le BI90 +® de Broockhaven Instrument Corporation.

Au sens de la présente invention, les particules interférentielles comprennent au moins les nacres de type mica/oxyde d'étain/oxyde de titane telles que par exemple celles commercialisées sous les dénominations TIMIRON SILK BLUE®, TIMIRON SILK RED®, TIMIRON SILK GREEN®, TIMIRON SILK GOLD® et TIMIRON SUPER SILK® proposées par la société MERCK et les nacres mica/oxyde de fer/oxyde de titane telles que par exemple les FLAMENCO SATIN BLUE®, FLAMENCO SATIN RED® et FLAMENCO SATIN VIOLET® proposées par la société ENGELHARD et leurs mélanges.

Il est entendu que le choix de ces particules interférentielles est opéré de manière à être par ailleurs compatible avec les exigences en terme de clarté et de saturation requises pour les compositions selon l'invention. D'une manière générale, ces particules interférentielles sont présentes en quantité suffisante pour obtenir un effet homogène en terme de coloration tout en préservant la carnation naturelle de la peau et/ou des lèvres.

Les particules sont présentes en une quantité inférieure à 7 %, et plus particulièrement inférieure à 5 %, et mieux encore allant de 2 à 5 % en poids par rapport au poids total de la composition.

**Charges**

Comme précisé précédemment, la présence d'une charge en association avec des particules interférentielles est avantageuse dans la mesure où elle contribue à procurer une homogénéisation spatiale améliorée du reflet procuré par ces particules.

D'une manière générale, le reflet d'une particule interférentielle n'est observable que pour certains angles d'observation, dits encore angles de réflexion angulaire. En conséquence, la répartition angulaire du reflet coloré est étroite et la correction colorielle apportée par l'utilisation de ces particules est donc inhomogène dans sa répartition spatiale.

De manière inattendue, les inventeurs ont constaté qu'en associant des charges matifiantes, à des particules interférentielles particulières de petites tailles, il était possible d'accroître la répartition angulaire du reflet coloré procuré par ces particules interférentielles.

Cet effet peut notamment être évalué selon la technique suivante.

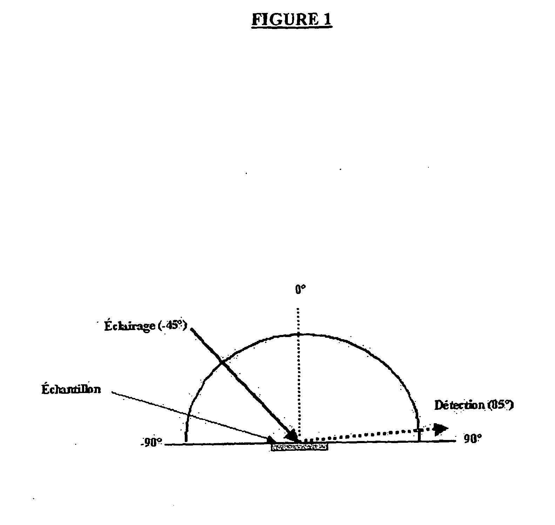

- Dans le cas d'une composition fluide, la composition à tester est étalée sous la forme d'un film dont l'épaisseur mesurée au moment du dépôt, c'est-à-dire avant séchage à l'air, est de 30 $\mu$m sur une carte de contraste ERICHSEN type 24/5 au moyen d'un applicateur automatique de chez BRAIVE INSTRUMENTS. L'étalement est ensuite disposé dans une étuve thermostatée et ventilé 24 heures à 37 °C. Les mesures sont réalisées sur le fond noir de la carte de contraste à l'aide d'un spectrogonioréflectomètre GON 360® commercialisé par la société Instrument System selon la géométrie représentée en figure 1.

L'évolution de la réflectance est appréciée en fonction de l'angle d'observation à la longueur d'onde correspondant à la zone du spectre où la réflectance du type de particules interférentielles ou du mélange de plusieurs types de particules interférentielles considérés selon l'invention est la plus significative.

Dans le cas de compositions cosmétiques non fluides, l'application de ladite composition sur la carte de contraste peut être réalisée selon les consignes suivantes :

- Pour des poudres compactées, on procède à leur décompactage de manière à les transformer en poudres libres. On dépose la poudre de manière homogène sur une surface plane puis on applique sur la poudre un film plastique transparent adhésif avec une pression de 100 g/cm$^2$, de manière à ce que la poudre colle à l'adhésif et à obtenir une surface adhésive saturée de poudre. La face adhésive chargée de poudre est ensuite posée contre une plaque de verre transparente et l'ensemble est déposé sur la carte de contraste. Les mesures sont effectuées comme précédemment.
- Pour un stick, celui-ci peut être fondu de manière à être déposé sous la forme d'une couche de 20 $\mu$m d'épaisseur sur un film transparent. On laisse sécher 10 mn à 37 °C dans une étuve puis on applique le film sur la carte de contraste, à l'instar de ce qui a été décrit plus haut.
- Pour le cas d'un spray, on dépose une épaisseur de 20 $\mu$m de composition et on laisse sécher 10 mn à 37 °C dans une étuve.

Dans le cadre d'une composition selon l'invention, il est ainsi observé une homogénéisation de la couleur sous tous les angles d'observation lorsque les particules interférentielles sont associées à une charge.

Avantageusement, la charge considérée selon l'invention peut être choisie en termes de nature, taille particulaire et/ou quantité de manière à conférer à ladite composition, une variation de réflectance définie telle que d'une part la réflectance maximale mesurée au sommet du pic de réflexion est inférieure à 150 %, de préférence inférieure à 100 % et plus préférentiellement inférieure à 75 %, et d'autre part, la réflectance minimale mesurée à la base du pic de réflexion est supérieure à 5 %. Cette variation de réflectance est mesurée pour une plage d'angles d'observation variant de 0 à 80° et à la longueur d'onde correspondant à la zone du spectre où la réflectance des particules interférentielles associées est la plus importante.

Selon un mode de réalisation particulier, les matériaux utilisés à titre de charge possèdent une taille particulaire exprimée en volume comparable à celle des particules interférentielles.

Ainsi, les charges présentent avantageusement une taille volumique inférieure à 40 $\mu$m, notamment allant de 0,5 à 40 $\mu$m, en particulier inférieure à 30 $\mu$m, voire inférieure à 15 $\mu$m et mieux allant de 1 à 15 $\mu$m.

Les charges considérées sont des charges dites matifiantes, c'est-à-dire des charges présentant un indice de réfraction inférieur ou égal à 2,2, notamment inférieur ou égal à 2, en particulier inférieur ou égal à 1,8, et plus particulièrement variant de 1,3 à 1,6.

L'indice de réfraction des particules peut être évalué par la méthode dite d'effacement de contraste. En choisissant deux solvants totalement miscibles d'indices de réfraction relativement éloignés (l'éthanol : 1,36 et alcool phényléthylique : 1,529), il est possible de réaliser des mélanges possédant des indices de réfraction intermédiaires. Les particules en question sont mises en suspension dans ces différents mélanges de solvant et la transparence de ces solutions est ensuite évaluée à l'aide d'un turbidimètre Hach 2100P® commercialisé par la société HACH. L'indice de réfraction de la particule est égal à celui du mélange de solvants pour lequel on obtient la solution la moins turbide, c'est-à-dire présentant le trouble le plus faible et qui correspond à l'écart d'indice de réfraction minimal entre les particules et le mélange de solvant.

Le pouvoir matifiant des compositions les contenant peut être caractérisé à l'aide du protocole suivant.

La composition à tester est étalée à raison de 2 mg/cm$^2$ sur une carte de contraste (PRUFKARTE type 24/5-250 cm$^2$ commercialisée par la société ERICHSEN) à l'aide d'un tire-film mécanique. La composition est ensuite séchée une nuit à une température de 37 °C préalablement à la mesure de sa réflexion à l'aide d'un gonioréflectomètre commercialisé par la société MICROMODULE. Le résultat obtenu est le rapport R entre la réflexion spéculaire et la réflexion diffuse. La valeur de R est d'autant plus faible que l'effet matifiant procuré par la charge est important.

Dans le cadre de la présente invention, la charge matifiante considérée est choisie de telle manière qu'elle confère à ladite composition un pouvoir matifiant inférieur à 0,8, notamment inférieur à 0,75, et de préférence allant de 0,15 à 0,75.

Avantageusement, les charges matifiantes présentent une taille volumique inférieure à 40 $\mu$m, notamment allant de 0,5 à 40 $\mu$m, et mieux de 1 à 15 $\mu$m.

Conviennent à l'invention, à titre de charges à pouvoir matifiant, les charges choisies parmi :

- les microparticules poreuses de silice, comme par exemple les SILICA BEADS SB150® et SB700® de MIYOSHI de taille moyenne de 5 $\mu$m et les SUNSPHERES Série-H® d'ASAHI GLASS comme par exemple les SUNSPHERE H33®, H51® et H53® de tailles respectives de 3, 5 et 5 $\mu$m ;
- les particules hémisphériques creuses de silicone comme par exemple les NLK 500®, NLK 506® et NLK 510® de TAKEMOTO OIL AND FAT ;
- et leurs mélanges.

En ce qui concerne plus particulièrement les particules hémisphériques creuses de silicone, il peut s'agir de portions de sphères creuses en forme de « bols ». Celles-ci peuvent être obtenues comme décrit dans la demande JP-2003 128 788. Des portions de sphères creuses en forme de fer à cheval sont aussi décrites dans la demande JP-A-2000-191 789.

Ces particules peuvent notamment être les particules dites méthyle silanol/silicate crosspolymer comme les dénominations NLK 500, NLK 506 et NLK 510.

A titre illustratif des autres charges convenant à l'invention, on peut notamment citer le talc, le mica, le kaolin, la silice colloïdale, la poly-$\beta$-alanine, le polyéthylène, la lauroyl-lysine, l'amidon, le nitrure de bore, les poudres micronisées de polytétrafluoroéthylène, le carbonate de calcium précipité, le carbonate et l'hydrocarbonate de magnésium, le sulfate de baryum, l'hydroxyhapatite, les microcapsules de verre ou de céramique et les savons métalliques dérivés d'acides organiques carboxyliques ayant de 8 à 22 atomes de carbone, notamment de 12 à 18 atomes de carbone, par exemple le stéarate de zinc, de magnésium ou de lithium, le laurate de zinc, le myristate de magnésium et leurs mélanges.

La charge est présente à raison de moins de 10 %, et mieux allant de 1 à 10 % en poids, et en particulier à raison de moins de 5 % en poids et mieux encore allant de 2 à 5 % en poids par rapport au poids total de la composition.

La quantité en charge est ajustée de manière à ce que le rapport pondéral charge/particules interférentielles est supérieur ou égal à 0,8, par exemple varie de 0,8 à 2,5.

**Milieu physiologiquement acceptable**

Les particules interférentielles et les charges selon l'invention sont conditionnées dans un milieu physiologiquement acceptable constituant le support de la composition cosmétique.

Par "milieu physiologiquement acceptable", on désigne un milieu non toxique et susceptible d'être appliqué sur la peau et/ou les lèvres d'êtres humains. Le milieu physiologiquement acceptable est généralement adapté à la nature de la peau sur laquelle doit être appliquée la composition ainsi qu'à la forme sous laquelle la composition est destinée à être conditionnée, notamment fluide ou non fluide à la température ambiante et sous pression atmosphérique.

Ainsi, les compositions selon l'invention peuvent être formulées sous une forme fluide ou solide de type poudre, libre, compacte ou coulée.

Selon une variante de l'invention, elles comprennent au moins une phase aqueuse, et en particulier de l'eau associée le cas échéant à une phase grasse.

## Phase aqueuse

La composition selon l'invention peut comprendre au moins un milieu aqueux, constituant une phase aqueuse, qui peut former la phase continue de la composition considérée.

Cette phase aqueuse peut être constituée en tout ou partie et plus particulièrement est constituée essentiellement d'eau.

Elle peut également comprendre un mélange d'eau et de solvant organique miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, et les aldéhydes en $C_2$-$C_4$.

La phase aqueuse (eau et éventuellement le solvant organique miscible à l'eau) peut être présente, à une teneur allant de 1 % à 95 % en poids, notamment allant de 3 % à 80 % en poids, et en particulier allant de 5 % à 60 %, en poids par rapport au poids total de la composition considérée.

Un tel milieu peut comprendre en outre une huile volatile telle que définie ci-après.

La composition selon l'invention peut également posséder par exemple une phase grasse continue, pouvant contenir moins de 5 % d'eau, notamment moins de 1 % d'eau par rapport à son poids total et en particulier être sous forme anhydre.

## Phase grasse

La phase grasse peut notamment comporter au moins un corps gras liquide à température ambiante (25 °C) et/ou un corps gras solide à température ambiante tel que les cires, les corps gras pâteux, les gommes et leurs mélanges. La phase grasse peut en outre contenir des solvants organiques lipophiles.

La phase grasse de la composition selon l'invention peut notamment comprendre, à titre de corps gras liquide, au moins une huile volatile ou non volatile ou un de leurs mélanges.

Par "huile volatile", on entend au sens de l'invention toute huile susceptible de s'évaporer au contact de la peau en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,01 à 300 mm de Hg (1,33 Pa à 40.000 Pa) et de préférence supérieure à 0,3 mm de Hg (30 Pa).

Par "huile non volatile", on entend une huile restant sur la peau à température ambiante et pression atmosphérique au moins plusieurs heures et ayant notamment une pression de vapeur inférieure à 0,01 mm de Hg (1,33 Pa).

Ces huiles volatiles ou non volatiles peuvent être des huiles hydrocarbonées, des huiles siliconées, ou leurs mélanges. On entend par "huile hydrocarbonée", une huile contenant principalement des atomes d'hydrogène et de carbone et éventuellement des atomes d'oxygène, d'azote, de soufre, de phosphore.

Les huiles hydrocarbonées volatiles peuvent être choisies parmi les huiles hydrocarbonées ayant de 8 à 16 atomes de carbone, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars® ou de Permetyls®, les esters ramifiés en $C_8$-$C_{16}$ tels que le néopentanoate d'iso-hexyle, et leurs mélanges. D'autres huiles hydrocarbonées volatiles comme les distillats de pétrole, notamment ceux vendus sous la dénomination Shell Solt® par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité ≤ 8 centistokes (8 x $10^{-6}$ m$^2$/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile peut être présente dans une composition selon l'invention à une teneur allant de 0,1 à 98 % en poids, notamment de 1 % à 65 % en poids, et en particulier de 2 % à 50 % en poids, par rapport au poids total de la composition.

Les huiles non volatiles peuvent notamment être choisies parmi les huiles hydrocarbonées fluorées et/ou siliconées non volatiles.

Comme huile hydrocarbonée non volatile, on peut notamment citer :

- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, d'amande douce, de palme, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810, 812 et 818® par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges,
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit $\geq 10$, comme par exemple l'huile de Purcellin (octanoate de cétostéaryle), le myristate d'isopropyle, le palmitate d'isopropyle, les benzoates d'alcools en $C_{12}$ à $C_{15}$, le laurate d'hexyle, l'adipate de diisopropyle, l'isononanoate d'isononyle, le palmitate de 2-éthyl-hexyle, l'isostéarate d'isostéaryle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol,
- les acides gras supérieurs tels que l'acide oléique, l'acide linoléique, l'acide linolénique et leurs mélanges.
  Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates.
  Les huiles non volatiles peuvent être présentes dans une composition selon l'invention en une teneur allant de 0,01 à 90 % en poids, notamment de 0,1 à 85 % en poids, et en particulier de 1 % à 70 % en poids, par rapport au poids total de la composition.
  Plus généralement, le corps gras liquide peut être présent à raison de 0,01 à 90 % en poids et notamment de 0,1 à 85 % en poids, par rapport au poids de la phase grasse.
  En ce qui concerne le corps gras solide à température ambiante et à pression atmosphérique, il peut être choisi parmi les cires, les corps gras pâteux, les gommes et leurs mélanges. Ce corps gras solide peut être présent à raison de 0,01 à 50 %, notamment de 0,1 à 40 % et en particulier de 0,2 à 30 % en poids, par rapport au poids total de la phase grasse.

**Agents tensioactifs**

La composition selon l'invention peut en outre contenir des agents tensioactifs émulsionnants et co-émulsionnants présents notamment en une proportion allant de 0,1 à 30 % en poids, et mieux de 5 % à 15 % en poids, par rapport au poids total de la composition.

Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Comme tensioactif utilisable dans l'invention, adapté à l'obtention d'une émulsion E/H, on peut citer notamment les diméthicone copolyols tels que le mélange de cyclométhicone et de diméthicone copolyol, vendu sous la dénomination « DC 5225 C » par la société Dow Corning, et les diméthicone copolyols tels que le laurylmethicone copolyol vendu sous la dénomination Dow Corning 5200 Formulation Aid® par la société Dow Corning et le Cetyl diméthicone copolyol vendu sous la dénomination ABIL EM 90R® par la société Goldschmidt, ou le mélange polyglycéryl-4 isostéarate/cétyl diméthicone copolyol/hexyllaurate vendu sous la dénomination ABIL WE 09® par la société Goldschmidt.

On peut y ajouter un ou plusieurs co-émulsionnants, qui peut, de manière avantageuse, être choisi dans le groupe comprenant les esters alkylés de polyol. Comme esters alkylés de polyol, on peut citer notamment les esters de glycérol et/ou de sorbitan et par exemple l'isostéarate de polyglycérol, tel que le produit commercialisé sous la dénomination Isolan GI 34® par la société Goldschmidt, l'isostéarate de sorbitan, tel que le produit commercialisé sous la dénomination Arlacel 987® par la société ICI, l'isostéarate de sorbitan et de glycérol, tel que le produit commercialisé sous la dénomination Arlacel 986® par la société ICI, et leurs mélanges.

Comme émulsifiant adapté à l'obtention d'une émulsion E/H, conviennent notamment les tensioactifs polyisobutylene à terminaison succinique estérifiée, tels que ceux commercialisés sous les noms de Lubrizol 5603® et Chemcinnate 2000® par les sociétés Lubrizol et Chemron.

On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21® par la société Shin Etsu.

Comme tensioactif utilisable dans l'invention, adapté à l'obtention d'une émulsion H/E, on peut citer par exemple les tensioactifs non ioniques, et notamment les esters de polyols et d'acide gras à chaîne saturée ou insaturée comportant par exemple de 8 à 24 atomes de carbone et mieux de 12 à 22 atomes de carbone, et leurs dérivés oxyalkylénés, c'est-à-dire comportant des unités oxyéthylénés et/ou oxypropylénés, tels les esters de glycéryle et d'acide gras en $C_8$-$C_{24}$, et leurs dérivés oxyalkylénés ; les esters de polyéthylène glycol et d'acide gras en $C_8$-$C_{24}$, et leurs dérivés oxyalkylénés ; les esters de sorbitol et d'acide gras en $C_8$-$C_{24}$, et leurs dérivés oxyalkylénés ; les esters de sucre (sucrose, glucose, alkylglucose) et d'acide gras en $C_8$-$C_{24}$, et leurs dérivés oxyalkylénés ; les éthers d'alcools gras; les éthers de sucre et d'alcools gras en $C_8$-$C_{24}$; les éthers oxyéthylénés d'acide gras et de glucose ou d'alkylglucose; et leurs mélanges.

Comme ester de glycéryle et d'acide gras, on peut citer notamment le stéarate de glycéryle (mono-, di- et/ou tri-stéarate de glycéryle) (nom CTFA : glycéryl stéarate) ou le ricinoléate de glycéryle, et leurs mélanges.

Comme ester de polyéthylène glycol et d'acide gras, on peut citer notamment le stéarate de polyéthylène glycol (mono-, di- et/ou tri-stéarate de polyéthylène glycol), et plus spécialement le monostéarate de polyéthylène glycol 50 OE (nom CTFA : PEG-50 stéarate), le monostéarate de polyéthylène glycol 100 OE (nom CTFA : PEG-100 stéarate) et leurs mélanges.

On peut aussi utiliser des mélanges de ces tensioactifs, comme par exemple le produit contenant du Glyceryl stéarate et du PEG-100 stéarate, commercialisé sous la dénomination ARLACEL 165® par la société Uniquema, et le produit contenant du Glyceryl stearate (mono-distéarate de glycéryle) et du stéarate de potassium, commercialisé sous la dénomination TEGIN® par la société Gold-schmidt (nom CTFA : glyceryl stearate SE).

Comme ester d'acide gras et de glucose ou d'alkylglucose, on peut citer en particulier le palmitate de glucose, les sesquistéarates d'alkylglucose comme le sesquistéarate de méthylglucose, les palmitates d'alkylglucose comme le palmi-tate de méthylglucose ou d'éthylglucose, les esters gras de méthylglucoside et plus spécialement le diester de méthylglucoside et d'acide oléique (nom CTFA : Methyl glucose dioleate) ; l'ester mixte de méthylglucoside et du mélange acide oléique / acide hydroxystéarique (nom CTFA : Methyl glucose dio-leate/hydroxystearate) ; l'ester de méthylglucoside et d'acide isostéarique (nom CTFA : Methyl glucose isostearate) ; l'ester de méthylglucoside et d'acide lauri-que (nom CTFA : Methyl glucose laurate) ; le mélange de monoester et de dies-ter de méthylglucoside et d'acide isostéarique (nom CTFA : Methyl glucose sesqui-isostearate) ; le mélange de monoester et de diester de méthylglucoside et d'acide stéarique (nom CTFA : Methyl glucose sesquistearate) et en particulier le produit commercialisé sous la dénomination Glucate SS® par la société AMERCHOL, et leurs mélanges.

Comme éthers oxyéthylénés d'acide gras et de glucose ou d'alkylglucose, on peut citer par exemple les éthers oxyéthylénés d'acide gras et de méthylglucose, et en particulier l'éther de polyéthylène glycol de diester de méthyl glucose et d'acide stéarique à environ 20 moles d'oxyde d'éthylène (nom CTFA : PEG-20 methyl glucose distearate) tel que le produit commercialisé sous la dé-nomination Glucam E-20 distearate® par la société AMERCHOL ; l'éther de poly-éthylène glycol du mélange de monoester et de diester de méthyl glucose et d'acide stéarique à environ 20 moles d'oxyde d'éthylène (nom CTFA : PEG-20 methyl glucose sesquistearate) et en particulier le produit commercialisé sous la dénomination Glucamate SSE-20® par la société AMERCHOL et celui commer-cialisé sous la dénomination Grillocose PSE-20® par la société GOLDSCHMIDT, et leurs mélanges.

Comme esters de sucrose, on peut citer par exemple le palmito-stéarate de saccharose, le stéarate de saccharose et le mono laurate de saccharose.

Comme éthers d'alcools gras, on peut citer par exemple les éthers de polyéthylène glycol et d'alcool gras comportant de 8 à 30 atomes de carbone, et notamment de 10 à 22 atomes de carbone, tels que les éthers de polyéthylène glycol et d'alcools cétylique, stéarylique, cétéarylique (mélange d'alcools cétylique et stéarylique). On peut citer par exemple les éthers comportant de 1 à 200 et de préférence de 2 à 100 groupes oxyéthylénés, tels que ceux de nom CTFA Ceteareth-20, Ceteareth-30, et leurs mélanges.

Comme éthers de sucre, on peut citer notamment les alkylpolyglucosides, et par exemple le decylglucoside comme le produit commercialisé sous la déno-mination MYDOL 10® par la société Kao Chemicals, le produit commercialisé sous la dénomination PLANTAREN 2000® par la société Henkel, et le produit commercialisé sous la dénomination ORAMIX NS 10® par la société Seppic ; le caprylyl/capryl glucoside comme le produit commercialisé sous la dénomination ORAMIX CG 110 par la Société Seppic ou sous la dénomination LUTENSOL GD 70® par la Société BASF ; le laurylglucoside comme les produits commercialisés sous les dénominations PLANTAREN 1200 N® et PLANTACARE 1200® par la société Henkel ; le coco-glucoside comme le produit commercialisé sous la dénomination PLANTACARE 818/UP® par la société Henkel ; le cétostéaryl glucoside éventuellement en mélange avec l'alcool cétostéarylique, commercialisé par exemple sous la dénomination MONTANOV 68® par la société Seppic, sous la dénomination TEGO-CARE CG90® par la société Goldschmidt et sous la dé-nomination EMULGADE KE3302® par la société Henkel ; l'arachidyl glucoside, par exemple sous la forme du mélange d'alcools arachidique et béhénique et d'arachidyl glucoside commercialisé sous la dénomination MONTANOV 202® par la société Seppic ; le cocoyléthylglucoside, par exemple sous la forme du mélange (35/65) avec les alcools cétylique et stéarylique, commercialisé sous la dénomination MONTANOV 82® par la société Seppic et leurs mélanges.

La composition selon l'invention peut également contenir en tant qu'émulsionnant ou co-émulsionnant une quantité avantageuse de polymères amphiphiles.

On entend par polymère amphiphile, tous polymères comportant à la fois une partie hydrophile et une partie hydrophobe et ayant la propriété de former un film séparant deux liquides de polarité différente et permettant ainsi de stabiliser des dispersions liquide

- liquide de type direct, inverse ou multiple. Les polymères amphiphiles convenant plus particulièrement réduisent la tension interfaciale eau/ huile jusqu'à 10 mN/m, et ce quelque soit l'huile. Ces polymères sont ioniques (anioniques ou cationiques) ou amphotères. Ils peuvent être hydrosolubles ou hydrodispersibles. On entend par hydrosoluble le fait qu'ils puissent se disperser dans l'eau sous forme de solution moléculaire. On entend par hydrodispersible le fait qu'ils puissent se disperser dans l'eau sous forme particulaire.

Les polymères amphiphiles conformes à l'invention ont généralement un poids moléculaire moyen en nombre allant de 1000 à 20 000 000 g/mole, de préférence allant de 20 000 à 8 000 000 et plus préférentiellement encore de 100 000 à 700 000 g/mole. Les quantités de polymères amphiphiles utilisées selon l'invention seront choisies de 0,01 à 20 %, de préférence de 0,1 à 10 % et plus préférentiellement encore de 0,2 % à 5 % en poids.

On peut utiliser plus particulièrement les copolymères acrylate/C10-C30-alkylacrylate tels que les produits vendus sous les noms PEMULEN TR1®, PEMULEN TR2® et CARBOBOL 1382® par la Société GOODRICH, ou bien leurs mélanges. On pourra utiliser aussi les copolymères acrylate/steareth-20 itaconate et copolymères acrylate/ceteth-20 itaconate vendus sous les noms STRUCTURE 2001® et STRUCTURE 3001® par la Société NATIONAL STARCH. Parmi les polymères amphiphiles réticulés ou non d'AMPS conviennent tout particulièrement les produits vendus sous les noms ARISTOFLEX LNC®, ARISTOFLEX SNC® et ARISTOFLEX HMS® par la Société CLARIANT.

A titre de terpolymères pouvant être utilisés, on peut citer le terpolymère acide méthacrylique/acrylate de méthyle/diméthyl m-isopropénylbenzylisocyanate d'alcool béhénylique éthoxylé à 40 OE, c'est-à-dire comportant 40 groupes oxyéthylénés vendus par la société AMERCHOL sous les dénominations VISCOPHOBE DB 1000 NP3-NP4®.

On peut également citer les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous la dénomination SALCARE SC 80®.

Les polymères anioniques utilisables selon l'invention sont par exemple les polymères d'acide isophtalique ou d'acide sulfoisophtalique, et en particulier les copolymères de phtalate/sulfoisophtalate/glycol (par exemple diéthylèneglycol/Phtalate/isophtalate/1,4-cyclohexanc-diméthanol) vendus sous les dénominations « Eastman AQ polymer » (AQ35S, AQ38S, AQ55S, AQ48 Ultra) par la société Eastman Chemical.

## Polymère filmogène

La composition selon l'invention peut comprendre, en outre, au moins un polymère filmogène.

Dans la présente demande, on entend par "polymère filmogène", un polymère apte à former à lui seul ou en présence d'un agent auxiliaire de filmification, un film continu et adhérent sur la peau et/ou les lèvres.

On utilise de préférence un polymère filmogène apte à former un film hydrophobe, c'est-à-dire un polymère dont le film a une solubilité dans l'eau à 25 °C inférieure à 1 % en poids.

Les compositions selon l'invention peuvent contenir en plus des adjuvants classiques tels que des colorants, des pigments, des azurants optiques, des parfums, des conservateurs, des filtres solaires physiques et chimiques, des séquestrants, des ajusteurs de pH (acides ou bases) et leurs mélanges. Bien entendu, le choix des adjuvants est effectué de manière à ne pas porter préjudice aux effets recherchés à travers l'application de la composition selon l'invention sur la peau et/ou les lèvres.

De façon connue, la composition cosmétique de l'invention peut également contenir des agents actifs habituels dans le domaine cosmétique. On peut citer en particulier tous les actifs connus pour leur activité sur le vieillissement de la peau comme les agents kératolytiques et prodesquamants, par exemple les $\alpha$-hydroxy-acides, les $\beta$-hydroxy-acides, les $\alpha$-céto-acides, les $\beta$-céto-acides, les rétinoïdes et leurs esters, le rétinal, l'acide rétinoïque et ses dérivés. On peut également citer les vitamines, telles que par exemple les vitamines B3, C, ou PP, B5, E, K1, et les dérivés de ces vitamines et notamment leurs esters ; les agents anti-radicaux libres, les filtres solaires ; les agents hydratant comme les polyols ; les céramides ; la DHEA et ses dérivés ; le coenzyme Q10 ; les agents blanchissants et dépigmentants comme par exemple l'acide kojique, les dérivés de para-aminophénols, l'arbutine et leurs dérivés, et leurs mélanges.

La composition selon l'invention se présente généralement sous la forme d'une formulation notamment à appliquer sur le visage ou le cou, d'un produit anti-cernes, d'un correcteur de teint, d'une crème ou base de maquillage pour le visage ou d'une composition de maquillage pour le corps ou les lèvres.

Plus particulièrement, il s'agit d'un produit de soin et/ou de maquillage destiné à la peau, notamment du visage.

La composition selon l'invention, peut se présenter sous une forme solide, par exemple pulvérulente, compactée ou coulée ou sous forme stick ou sous la forme d'un fluide par exemple pâteux ou liquide.

Avantageusement, elle se présente sous une forme fluide de type pâte souple, onguent, pommade solide ou fluide comme une crème. Par exemple, elle peut être un sérum (solution aqueuse épaissie), un gel aqueux ou une émulsion huile-dans-eau ou eau-dans-huile, mais aussi une émulsion multiple huile-eau-huile, glycol-huile-eau ou eau-huile-eau.

La composition peut être fabriquée par les procédés connus, généralement utilisés dans le domaine cosmétique.

Les exemples figurant ci-après sont présentés à titre illustratif et non limitatif du domaine de l'invention.

## Exemples

L'ensemble des compositions cosmétiques ci-après est préparé selon le protocole suivant :

On chauffe la phase B à environ 75 °C et on y incorpore le polyacryldiméthyltauramide d'ammonium. On agite jusqu'à l'obtention d'un gel homogène.
On chauffe la phase A à 75 °C.
On réalise l'émulsion en incorporant la phase A dans la phase B.
A 40-45 °C, on incorpore la phase C et on maintient l'agitation jusqu'à refroidissement complet.

## Exemple 1

composition cosmétique témoin

| | | |
|---|---|---|
| A - | Glycéryl stéarate (et) PEG-100 stéarate (ARLACEL 165 FL® commercialisé par UNIQUEMA) | 2,00 g |
| | Dimyristyl tartrate (et) cétéaryl alcool (et) C12-15 pareth-7 (et) PPG-25 laureth-25 (COSMACOL PSE® commercialisé par SASOL) | 1,50 g |
| | Cyclohexasiloxane | 10,00 g |
| | Alcool stéarylique | 1,00 g |
| B - | Eau | 80,80g |
| | Phénoxyéthanol | 1,00 g |
| | Polyacryldiméthyltauramide d'ammonium commercialisé par Clariant sous le nom Hostacerin AMPS® | 0,40 g |
| | Gomme xanthane | 0,20 g |
| C - | Nacre mica/oxyde de titane/oxyde d'étain (TIMIRON SILK BLUE® commercialisé par la société MERCK) | 3,00 g |

**Exemple 2**

Composition cosmétique selon l'invention

| A - | Glycéryl stéarate (et) PEG-100 stéarate | |
| | (ARLACEL 165 FL® commercialisé par UNIQUEMA) | 2,00 g |
| | Dimyristyl tartrate (et) cétéaryl alcool (et) C12-15 pareth-7 (et) PPG-25 laureth-25 | |
| | (COSMACOL PSE® commercialisé par SASOL) | 1,50 g |
| | Cyclohexasiloxane | 10,00 g |
| | Alcool stéarylique | 1,00 g |
| | | |
| B - | Eau | 75,75 g |
| | Phénoxyéthanol | 1,00 g |
| | Pentasodium éthylène diamine tétraméthylène phosphate | 0,05 g |
| | Polyacryldiméthyltauramide d'ammonium commercialisé par Clariant sous le nom Hostacerin AMPS® | 0,40 g |
| | Gomme xanthane | 0,20 g |
| | | |
| C - | Nacres mica/oxyde de titane/oxyde d'étain | |
| | (TIMIRON SILK BLUE® commercialisé par la société MERCK) | 3,00 g |
| | Particules hémisphériques creuses de silicone (NLK 510 commercialisées par la société TAKEMOTO OIL AND FATS) | 5,00 g |

Les valeurs colorimétriques de clarté et de saturation mesurées selon le protocole décrit précédemment à l'aide d'un colorimétrique CHROMAMETER CR400® de MINOLTA pour la composition de l'exemple 2 selon l'invention sont les suivantes :

$$L = 86,46 +/- 0.04, \ C^* = 1,71 +/- 0.01.$$

L'effet bénéfique de l'association charge/particules interférentielles a été vérifié, comparativement à la composition témoin de l'exemple 1, par mesure de la variation de réflectance à la longueur d'onde correspondant à la zone du spectre où la réflectance de la nacre Timiron Silk Blue® est la plus importante, c'est-à-dire à 425 nm, selon la méthode décrite précédemment.

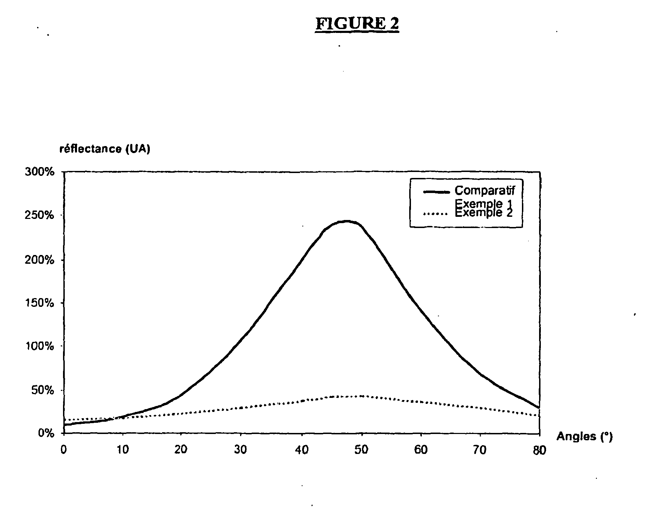

Les résultats sont présentés graphiquement en figure 2. On note que la composition témoin possède une forte saturation à la direction spéculaire (40-50°) alors que la composition selon l'invention présente une répartition angulaire de la réflectance à 425 nm beaucoup plus monotone tout en restant non nulle quelque soit l'angle d'observation.

L'association conforme à l'invention permet donc une homogénéisation de la couleur sous les angles.

**Revendications**

1. Composition cosmétique pour le maquillage et/ou le soin de la peau et/ou des lèvres comprenant, dans un milieu physiologiquement acceptable, au moins des particules interférentielles dont la taille moyenne en volume est inférieure à 40 μm comprenant au moins des nacres de type mica/oxyde d'étain/oxyde de titane et/ou de type mica/oxyde de fer/oxyde de titane et au moins une charge à pouvoir matifiant choisie parmi des microparticules poreuses de silice, et des particules hémisphériques creuses de silicone et leurs mélanges, lesdites particules interférentielles étant présentes en une quantité inférieure à 7 % en poids par rapport au poids total de la composition, ladite charge étant présente à raison de moins de 10 % en poids par rapport au poids total de la composition, ladite charge étant en une quantité telle que le rapport pondéral charge/particules interférentielles soit supérieur ou égal à 0,8, ladite charge étant apte à conférer à ladite composition un pouvoir matifiant inférieur à 0,8, avec ladite composition possédant en outre une clarté L* supérieure à 60 et une saturation C* inférieure à 10.

**2.** Composition selon la revendication précédente, **caractérisée en ce que** ladite charge est en une quantité telle que le rapport pondéral charge/particules interférentielles varie de 0,8 à 2,5.

**3.** Composition selon la revendication 1 ou 2, dans laquelle ladite charge confère à ladite composition une variation de réflectance, mesurée pour une plage d'angles d'observation variant de 0 à 80° et à la longueur d'onde correspondant à la zone du spectre où la réflectance des particules interférentielles associées est la plus importante, telle que d'une part, la réflectance maximale, mesurée, au sommet du pic de réflexion, est inférieure à 150 %, et la réflectance minimale, mesurée à la base du pic de réflexion, est supérieure à 5 %.

**4.** Composition selon l'une quelconque des revendications 1 à 3, **caractérisée en ce qu'**elle possède une saturation C* inférieure à 5.

**5.** Composition selon l'une quelconque des revendications 1 à 4, **caractérisée en ce qu'**elle possède une clarté L* supérieure à 75.

**6.** Composition selon la revendication 3, **caractérisée par le fait que** la réflectance maximale est inférieure à 100 % et en particulier inférieure à 75 %.

**7.** Composition selon l'une quelconque des revendications précédentes, **caractérisée en ce qu'**elle possède une clarté L* supérieure à 80 et en particulier supérieure à 86.

**8.** Composition selon l'une quelconque des revendications précédentes, **caractérisée en ce qu'**elle possède une saturation C* inférieure à 3 et en particulier inférieure à 2.

**9.** Composition selon l'une- quelconque des revendications précédentes, **caractérisée en ce que** les particules interférentielles possèdent une taille moyenne en volume inférieure à 30 $\mu$m et plus particulièrement inférieure à 20 $\mu$m.

**10.** Composition selon l'une quelconque des revendications précédentes, **caractérisée en ce que** ladite charge possède une taille particulaire moyenne en volume équivalente à celle des particules interférentielles.

**11.** Composition selon l'une quelconque des revendications précédentes, **caractérisée en ce que** ladite charge possède un indice de réfraction inférieur ou égal à 2,2, notamment inférieur ou égal à 2, en particulier inférieur ou égal à 1,8 et plus particulièrement variant de 1,3 à 1,6.

**12.** Composition selon l'une quelconque des revendications précédentes, **caractérisée en ce que** ladite charge possède un pouvoir matifiant tel qu'elle confère à ladite composition un pouvoir matifiant inférieur à 0,75.

**13.** Composition selon l'une quelconque des revendications précédentes, **caractérisée en ce qu'**elle comprend en outre à titre de charge le talc, le mica, le kaolin, la silice colloïdale, la poly-β-alanine, le polyéthylène, la lawoyl-lysine, l'amidon, le nitrure de bore, le carbonate de calcium précipité, le carbonate et l'hydro-carbonate de magnésium, le sulfate de baryum, l'hydroxyhapatite, les microcapsules de verre ou de céramique et les savons métalliques dérivés d'acides organiques carboxyliques ayant de 8 à 22 atomes de carbone et leurs mélanges.

**14.** Composition selon l'une quelconque des revendications précédentes, **caractérisée en ce qu'**elle comprend au moins une phase aqueuse.

**15.** Composition selon l'une quelconque des revendications précédentes, **caractérisée en ce que** ladite composition comprend au moins une phase grasse.

**16.** Composition selon l'une quelconque des revendications précédentes, **caractérisée en ce qu'**elle comprend au moins un adjuvant choisi parmi les colorants, les pigments, les azurants optiques, les parfums, les conservateurs, les filtres solaires physiques et chimiques, les séquestrants, les ajusteurs de pH (acides ou bases) et leurs mélanges.

**17.** Composition selon l'une quelconque des revendications précédentes, **caractérisée en ce qu'**elle comprend en outre un polymère filmogène.

**18.** Composition selon l'une quelconque des revendications précédentes, **caractérisée en ce qu'**elle se présente sous forme d'une pâte fluide, d'un sérum, d'une émulsion directe, inverse ou multiple ou d'un gel aqueux.

**19.** Utilisation cosmétique d'une composition telle que définie précédemment pour obtenir un effet d'éclaircissement et/ou d'homogénéisation de la peau et/ou des lèvres.

**20.** Utilisation cosmétique d'une composition telle que définie en revendication 1 à 17 pour le camouflage de dyschromies cutanées.

**21.** Procédé de maquillage de la peau et/ou des lèvres comprenant au moins l'application sur ladite peau et/ou les lèvres d'une composition telle que définie en revendications 1 à 18.

**Claims**

**1.** Cosmetic composition for making up and/or caring for the skin and/or the lips, comprising, in a physiologically acceptable medium, at least interference particles with a volume-average size of less than 40 $\mu$m comprising at least nacres of mica/tin oxide/titanium oxide type and/or of mica/iron oxide/titanium oxide type and at least one filler with matting power chosen from porous silica microparticles and hemispherical hollow silicone particles and mixtures thereof, the said interference particles being present in an amount of less than 7% by weight relative to the total weight of the composition, the said filler being present in an amount of less than 10% by weight relative to the total weight of the composition, the said filler being in an amount such that the filler/interference particle weight ratio is greater than or equal to 0.8, the said filler being such that it gives the said composition a matting power of less than 0.8, the said composition also having a lightness L* of greater than 60 and a saturation C* of less than 10.

**2.** Composition according to the preceding claim, **characterized in that** the said filler is in an amount such that the filler/interference particle weight ratio ranges from 0.8 to 2.5.

**3.** Composition according to claim 1 or 2, wherein the said filler gives the said composition a variation in reflectance, measured for a range of angles of observation ranging from 0 to 80° and at a wavelength corresponding to the region of the spectrum in which the reflectance of the associated interference particles is the greatest, such that, firstly, the maximum reflectance measured at the top of the reflection peak is less than 150%, and the minimum reflectance measured at the base of the reflection peak is greater than 5%.

**4.** Composition according to any one of claims 1 to 3, **characterized in that** it has a saturation C* of less than 5.

**5.** Composition according to any one of claims 1 to 4, **characterized in that** it has a lightness L* of greater than 75.

**6.** Composition according to claim 3, **characterized in that** the maximum reflectance is less than 100% and in particular less than 75%.

**7.** Composition according to any one of the preceding claims, **characterized in that** it has a lightness L* of greater than 80 and in particular greater than 86.

**8.** Composition according to any one of the preceding claims, **characterized in that** it has a saturation C* of less than 3 and in particular less than 2.

**9.** Composition according to any one of the preceding claims, **characterized in that** the interference particles have a volume-average size of less than 30 $\mu$m and more particularly less than 20 $\mu$m.

**10.** Composition according to any one of the preceding claims, **characterized in that** the said filler has a volume-average particle size equivalent to that of the interference particles.

**11.** Composition according to any one of the preceding claims, **characterized in that** the said filler has a refractive index of less than or equal to 2.2, especially less than or equal to 2, in particular less than or equal to 1.8 and more particularly ranging from 1.3 to 1.6.

**12.** Composition according to any one of the preceding claims, **characterized in that** the said filler has a matting power such that it gives the said composition a matting power of less than 0.75.

**13.** Composition according to any one of the preceding claims, **characterized in that** it further comprises as filler talc, mica, kaolin, colloidal silica, poly-β-alanine, polyethylene, lauroyllysine, starch, boron nitride, precipitated calcium carbonate, magnesium carbonate, magnesium hydrogen carbonate, barium sulfate, hydroxyapatite, glass or ceramic microcapsules, and metal soaps derived from organic carboxylic acids containing from 8 to 22 carbon atoms, and mixtures thereof.

14. Composition according to any one of the preceding claims, **characterized in that** it comprises at least one aqueous phase.

15. Composition according to any one of the preceding claims, **characterized in that** the said composition comprises at least one fatty phase.

16. Composition according to any one of the preceding claims, **characterized in that** it comprises at least one adjuvant chosen from dyes, pigments, optical brighteners, fragrances, preserving agents, physical and chemical sunscreens, sequestrants and pH regulators (acids or bases), and mixtures thereof.

17. Composition according to any one of the preceding claims, **characterized in that** it also comprises a film-forming polymer.

18. Composition according to any one of the preceding claims, **characterized in that** it is in the form of a fluid paste, a serum, a direct, inverse or multiple emulsion, or an aqueous gel.

19. Cosmetic use of a composition as defined above, for obtaining a lightening and/or homogenizing effect of the skin and/or the lips.

20. Cosmetic use of a composition as defined in Claims 1 to 17 for covering skin dyschromia.

21. Process for making up the skin and/or the lips, comprising at least the application to the said skin and/or the lips of a composition as defined in Claims 1 to 18.

**Patentansprüche**

1. Kosmetische Zusammensetzung zum Schminken und/oder zur Pflege der Haut und/oder der Lippen, umfassend, in einem physiologisch annehmbaren Medium, mindestens Interferenzteilchen, deren mittlere Volumensgröße weniger als 40 μm beträgt und die umfassen: mindestens Perlmutte vom Typ Glimmer/Zinnoxid/Titanoxid und/oder vom Typ Glimmer/Eisenoxid/Titanoxid, und mindestens einen Füllstoff mit einem Mattierungsvermögen, der ausgewählt ist aus porösen Siliciumoxidmikroteilchen und hohlen halbkugelförmigenn Teilchen aus Silicon und ihren Mischungen, wobei die Interferenzteilchen in einer Menge von weniger als 7 Gew.-%, bezogen auf das Gesamtgewicht der Zusammensetzung, vorlieben, wobei der Füllstoff in einer Menge von weniger als 10 Gew.-%, bezogen auf das Gesamtgewicht Zusammensetzung, vorliegt, wobei der Füllstoff in einer solchen Menge vorliegt, dass das Gewichtsverhältnis Füllstoff/Interferenzteilchen größer als oder gleich 0,8 ist, wobei der Füllstoff der Zusammensetzung ein Mattierungsvermögen von weniger als 0,8 verleihen kann, wobei diese Zusammensetzung außerdem eine Helligkeit L* über 60 und eine Sättigung C* unter 10 besitzt.

2. Zusammensetzung nach dem vorhergehenden Anspruch, **dadurch gekennzeichnet, dass** der Füllstoff in einer solchen Menge vorliegt, dass das Gewichtsverhältnis Füllstoff/Interferenzteilchen von 0,8 bis 2,5 variiert.

3. Zusammensetzung nach Anspruch 1 oder 2, bei der der Füllstoff der Zusammensetzung eine solche Reflektanzänderung verleiht, gemessen für einen Bereich von Beobachtungswinkein von 0 bis 80° und bei der Wellenlänge, die der Zone des Spektrums entspricht, in der die Reflektanz der zugeordneten Interferenzteilchen am höchsten ist, dass einerseits die am Scheitel des Reflexionspeaks gemessene maximale Reflektanz kleiner als 150 % ist und die an der Basis des Reflexionspeaks gemessene minimale Reflektanz größer als 5 % ist.

4. Zusammensetzung nach einem der Ansprüche 1 bis 3, **dadurch gekennzeichnet, dass** sie eine Sättigung C* unter 5 besitzt.

5. Zusammensetzung nach einem der Ansprüche 1 bis 4, **dadurch gekennzeichnet, dass** sie eine Helligkeit L* über 75 besitzt.

6. Zusammensetzung nach Anspruch 3, **dadurch gekennzeichnet, dass** die maximale Reflektanz weniger als 100% und insbesondere weniger als 75% beträgt.

7. Zusammensetzung nach einem der vorhergehenden Ansprüche, **dadurch gekennzeichnet, dass** sie eine Helligkeit L* über 80 und insbesondere über 86 besitzt.

8. Zusammensetzung nach einem der vorhergehenden Ansprüche, **dadurch gekennzeichnet, dass** sie eine Sättigung C* unter 3 und insbesondere unter 2 besitzt.

9. Zusammensetzung nach einem der vorhergehenden Ansprüche, **dadurch gekennzeichnet, dass** die Interferenzteilchen eine mittlere Volumensgrößer unter 30 $\mu$m und insbesondere unter 20 $\mu$m besitzen.

10. Zusammensetzung nach einem der vorhergehenden Ansprüche, **dadurch gekennzeichnet, dass** der Füllstoff eine mittlere Volumensgröße der Teilchen besitzt, die mit der der Interferenzteilchen äquivalent ist.

11. Zusammensetzung nach einem der vorhergehenden Ansprüche, **dadurch gekennzeichnet, dass** der Füllstoff eine Brechungsindex kleiner als oder gleich 2,2, insbesondere kleiner als oder gleich 2, vor allem kleiner als oder gleich 1,8 und noch spezieller von 1,3 bis 1,6 besitzt.

12. Zusammensetzung nach einem der vorhergehenden Ansprüche, **dadurch gekennzeichnet, dass** der Füllstoff ein solches Mattierungsvermögen besitzt, dass er der Zusammensetzung ein Mattierungsvermögen unter 0,75 verleiht.

13. Zusammensetzung nach einem der vorhergehenden Ansprüche, **dadurch gekennzeichnet, dass** sie außerdem als Füllstoff umfasst: Talk, Glimmer, Kaolin, kolloidales Siliciumoxid, poly-□-Alanin, Polyethylen, Lauroyl Lysin, Stärke, Bornitrid, ausgefälltes Calciumcarbonat, Magnesiumcarbonat und -hydrocarbonat, Baryumsulfat, Hydroxyhapatit, Mikrokapseln aus Glas oder Keramik und Metallseifen, die von organischen Carbonsäuren mit 8 bis 22 Kohlenstoffatomen abgeleitet sind, und ihre Mischungen.

14. Zusammensetzung nach einem der vorhergehenden Ansprüche, **dadurch gekennzeichnet, dass** sie mindestens eine wässrige Phase umfasst.

15. Zusammensetzung nach einem der vorhergehenden Ansprüche, **dadurch gekennzeichnet, dass** die Zusammensetzung mindestens eine Fettphase umfasst.

16. Zusammensetzung nach einem der vorhergehenden Ansprüche, **dadurch gekennzeichnet, dass** sie mindestens einen Zusatz umfasst, der aus Farbstoffen, Pigmenten, optischen Aufhellern, Parfüms, Konservierungsmitteln, physikalischen und chemischen Sonnenfiltern, Komplexbildnern, pH-Einstellern (sauer oder basisch) und ihren Mischungen ausgewählt ist.

17. Zusammensetzung nach einem der vorhergehenden Ansprüche, **dadurch gekennzeichnet, dass** sie außerdem ein filmbildendes Polymer umfasst.

18. Zusammensetzung nach einem der vorhergehenden Ansprüche, **dadurch gekennzeichnet, dass** sie in Form einer fließfähigen Paste, eines Serums, einer direkten, inversen oder multiplen Emulsion oder eines wässrigen Gels vorliegt.

19. Kosmetische Verwendung einer Zusammensetzung, wie sie oben definiert ist, zum Erzeugen einer Wirkung der Aufhellung und/oder Homogenisierung der Haut und/oder der Lippen.

20. Kosmetische Verwendung einer Zusammensetzung, wie sie in den Ansprüche 1 bis 17 definiert ist, zum Abdecken von Hautdyschromien.

21. Verfahren zum Schminken der Haut und/oder der Lippen, umfassend mindestens das Auftragen einer Zusammensetzung, wie sie in den Ansprüche 1 bis 18 definiert ist, auf die Haut und/oder die Lippen.

## FIGURE 1

# FIGURE 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0151017 A
- US 5690916 A
- WO 04045524 A
- JP 2003128788 B
- JP 2000191789 A
- US 5412004 A
- US 5811487 A